(19) Europäisches Patentamt
European Patent Office
Office européen des brevets

(11) **EP 1 810 620 A1**

(12) **EUROPEAN PATENT APPLICATION**

(43) Date of publication:
**25.07.2007 Bulletin 2007/30**

(51) Int Cl.:
**A61B 8/08** (2006.01)

(21) Application number: **07001377.6**

(22) Date of filing: **23.01.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **24.01.2006 KR 20060007102**

(71) Applicant: **MEDISON CO., LTD.**
**Kangwon-do 250-870 (KR)**

(72) Inventors:
• **Jeong, Mok Kun**
**Seoul 139-768 (KR)**

• **Kwon, Sung Jae**
**Seoul 130-781 (KR)**
• **Yoon, Ra Young**
**Discusser & Medison Building**
**Seoul 135-280 (KR)**

(74) Representative: **Schmid, Wolfgang**
**Lorenz & Kollegen**
**Patent- und Rechtsanwaltskanzlei**
**Alte Ulmer Strasse 2**
**D-89522 Heidenheim (DE)**

(54) **Ultrasound diagnostic system and method of forming elastic images**

(57) There is provided an ultrasound diagnostic system, which includes: a unit for forming transmit signals, wherein waveforms of the transmit signals vary depending on whether or not a stress is applied to a target object; a probe for converting the transmit signals into ultrasound signals and forming receive signals based on ultrasound echo signals reflected from the target object in the presence and absence of the stress; a processor for computing a displacement and a strain of the target object due to the stress based on the receive signals; and a post-processing unit for forming an ultrasound elastic image based on the strain.

FIG. 2

**Description**

The present application claims priority from Korean Patent Application No. 10-2006-0007102 filed on January 24, 2006, the entire subject matter of which is incorporated herein by reference.

BACKGROUND

1. Field

The present invention generally relates to ultrasound diagnostic systems, and more particularly to an ultrasound diagnostic system and a method of forming ultrasound elastic images.

2. Background

An ultrasound diagnostic system has become an important and popular diagnostic tool since it has a wide range of applications. Specifically, the ultrasound diagnostic system has been extensively used in the medical profession due to its non-invasive and non-destructive nature.

The ultrasound diagnostic system generates and transmits ultrasound signals with a probe including an array of transducer elements. The ultrasound signals are generated when each transducer element is electrically excited and are transmitted to a target object such as a human body. As the ultrasound signals travel into the target object, ultrasound echo signals are produced on the surface of an internal structure in the target object. This is because the surface of the internal structure and a medium surrounding such structure have discontinuous acoustic impedance. As for the human body, the internal structure may be a group of tissues. The ultrasound echo signals return to the transducers and are then converted into electrical receive signals. A normal ultrasound image, such as a B-mode ultrasound image of the internal structure of the target object, is formed based on the receive signals.

If stress is applied to the target object, then the internal structure of the target object is deformed (that is, at least a portion of the internal structure is changed). The deformation of the internal structure causes shifts in the receive signals. An ultrasound elastic image is formed based on the shifts in the receive signals as well as the receive signals themselves.

Abnormal tissues such as a lesion (e.g., cancer or tumor) are harder than normal tissues. Further, the abnormal tissues are less deformed compared to the normal tissues when the stress is applied. In other words, the shift amount of the receive signals originating from the ultrasound echo signals at the abnormal tissues is less than that originating from the ultrasound echo signals at the normal tissues. Therefore, the abnormal tissues can be easily distinguished from the normal tissues in the ultrasound elastic image.

The ultrasound elastic image can be obtained by two methods, namely, an elastic coefficient imaging method and a strain imaging method. In the elastic coefficient imaging method, elastic coefficient of the tissue is adopted to form the ultrasound elastic image. In the strain imaging method, strain of the tissue with respect to the stress is adopted.

The shifts in the receive signals due to the stress are expressed as a position change of the tissue in the ultrasound elastic image. From such position change of the tissue, the elastic coefficient or the strain of the tissue can be obtained. Therefore, the position, size and condition of abnormal tissues can be diagnosed by reconstructing the elastic coefficient or the strain in one, two or three dimensions.

In case of one-dimensional deformation of medium, the relation of stress $\sigma$, elastic coefficient E and strain $\varepsilon$ can be expressed as equation (1).

$$\sigma = E\varepsilon \qquad \ldots\ldots\ldots(1)$$

From the equation (1), a profile of the elastic coefficient E or the strain s is obtained to form an ultrasound elastic image according to the elastic coefficient imaging method or the strain imaging method. In the strain imaging method, the stress is approximated to a constant, whereas the stress is estimated in the elastic imaging method since the stress cannot be directly measured. Therefore, it is difficult to utilize the elastic coefficient imaging method, which is a type of quantitative method. This is because an "inverse problem" needs to be solved in order to obtain the elastic coefficient E from the equation (1). For this reason, the strain imaging method, which is a type of qualitative method, has been studied since the early 1990's.

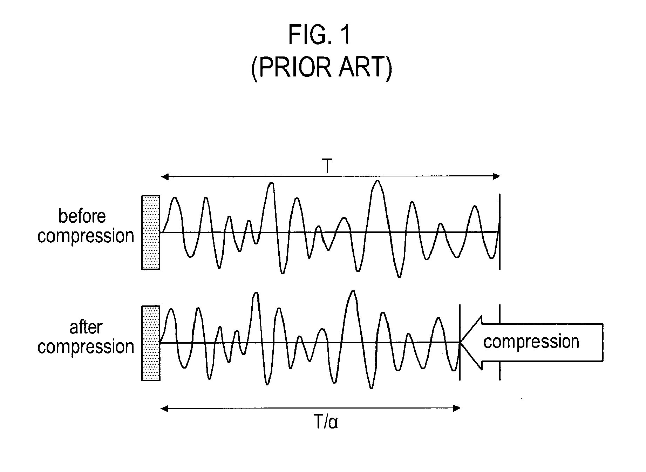

In order to obtain a strain of tissues, a first receive signal and a second receive signal, which are obtained without and with applying stress to a target object, respectively, are compared. As shown in FIG. 1, the second receive signal has a compression form of the first receive signal. In order to compare the first receive signal with the second receive signal, the second receive signal is extended to meet the same size of the target object without applying any stress, i.e., to obtain the effect of restoring spaces between reflectors in the target object under stress and those prior to applying stress.

However, conventionally, transmit signals having the same waveform are transmitted into the human body regardless of whether the stress is applied or not. Thus, a transmit signal applied to the human body under stress is also extended with restoring the spaces of the reflectors when the second receive signal is extended. The extension of the transmit signal undermines the estimation of the strain. Further, the correlation between the first and second receive signals is significantly degraded.

Hereinafter, the drawbacks of the conventional method will be described in detail. A receive signal $r(t)$ can be expressed with a transmit signal $p(t)$ for obtaining a B-mode ultrasound image and a scattering function $s(t)$ representing the positions of the reflectors, as shown in the following equation (2).

$$r(t) = p(t) * s(t) \qquad \dots\dots\dots (2)$$

In equation (2), the symbol "*" represents a convolution.

The scattering function $s(t)$ can be expressed with a reflection coefficient $A$ of the reflectors, a distance $d$ between a transducer element and the reflector, a sound speed $c$, and a total number $N$ of reflectors in the target object, as shown in the following equation (3).

$$s(t) = \sum_{i=1}^{N} A_i \delta\left(t - \frac{2d}{c}\right) = \sum_{i=1}^{N} A_i \delta(t - t_i) \qquad \dots\dots\dots (3)$$

When the same transmit signal $p(t)$ is used, the first receive signal $r_1(t)$ obtained without applying the stress and the second receive signal $r_2(t)$ obtained with applying the stress can be expressed by the following equations (4) and (5), respectively. In the equations (4) and (5), $S_1(t)$ and $S_2(t)$ are scattering functions under no stress and under stress, respectively.

$$r_1(t) = p(t) * s_1(t) \qquad \dots\dots\dots (4)$$

$$r_2(t) = p(t) * s_2(t) \qquad \dots\dots\dots (5)$$

When the compressibility of the second receive signal is denoted as $\alpha$, which is larger than 1, the scattering functions $S_1(t)$ and $S_2(t)$ have a relation as shown in the following equation (6), which is based on the equations (4) and (5).

$$s_2(t) = s_1(\alpha t) = s(\alpha t) \qquad \dots\dots\dots (6)$$

From the equation (6), it can be seen that the spaces between the reflectors decrease as the stress is applied.

In order to compute the strain by comparing the first receive signal $r_1(t)$ and the second receive signal $r_2(t)$, the two receive signals $r_1(t)$ and $r_2(t)$ are divided into a number of segments in a depth direction of the B-mode ultrasound image, respectively. A cross correlation of corresponding segments of the first and second receive signals, as well as a delay time at which the cross correlation has the maximum value, are obtained one after the other. The strain can be estimated with differentiating the cross correlation at the delay time.

The following equation (7) is obtained from the equations (5) and (6). Further, the following equation (8) is obtained by extending the equation (7) along the time axis.

$$r_2(t)=p(t)*s_1(\alpha t) \qquad \dots\dots\dots (7)$$

$$r_2(t/\alpha)=p(t/\alpha)*s_1(t) \qquad \dots\dots\dots (8)$$

As seen from the equation (8), if the second receive signal $r_2(t)$ is extended along the time axis, then the transmit signal $p(t)$ is also extended. Thus, even though the same transmit signal $p(t)$ is transmitted regardless of whether the stress is applied or not, the extended transmit signal $p(t/\alpha)$ influences the second receive signal $r_2(t)$. Therefore, it is difficult to accurately compute a maximum value of the cross correlation between the first receive signal $r_1(t)$ and $r_2(t/\alpha)$ obtained by extending the second receive signal $r_2(t)$. Moreover, there are a few tens of reflectors per unit wavelength in the human body. Thus, the waveforms of the first receive signal $r_1(t)$ and the second receive signal $r_2(t)$ become very different. Therefore, it is difficult to obtain the correlation between the first and second receive signals $r_1(t)$ and $r_2(t)$.

BRIEF DESCRIPTION OF THE DRAWINGS

Arrangements and embodiments may be described in detail with reference to the following drawings in which like reference numerals refer to like elements and wherein:
FIG. 1 is a graph showing a variation in wavelengths of the receive signals in the presence and absence of stress;
FIG. 2 shows an ultrasound diagnostic system constructed in accordance with one embodiment of the present invention;
FIG. 3 is a block diagram showing a probe of the ultrasound diagnostic system shown in FIG. 2; and
FIG. 4 shows an ultrasound diagnostic system constructed in accordance with another embodiment of the present invention.

DETAILED DESCRIPTION

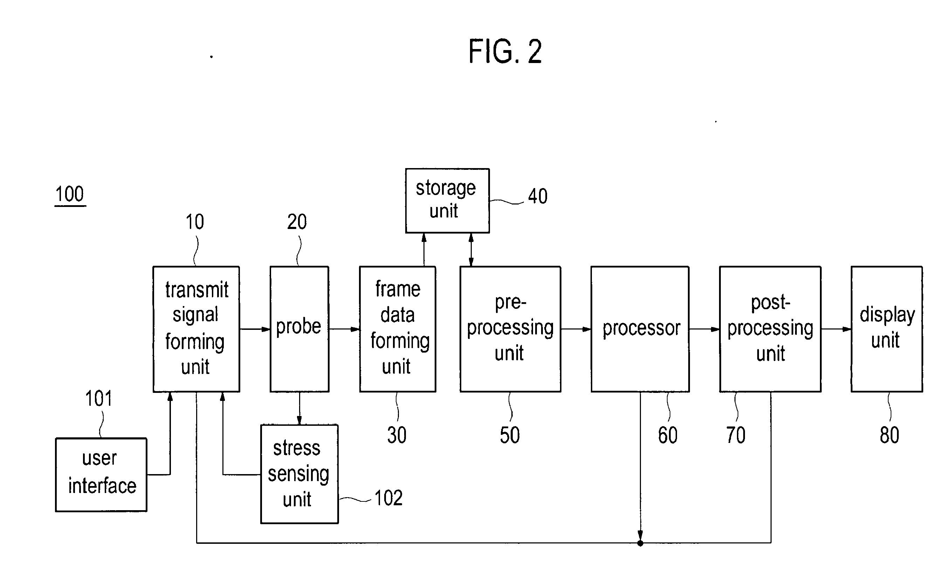

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.
One embodiment of the present invention will be described below with reference to the accompanying drawings. FIG. 2 illustrates an ultrasound diagnostic system 100 constructed in accordance with one embodiment of the present invention. The system 100 includes a transmit signal forming unit 10, a probe 20, a frame data forming unit 30, a storage unit 40, a pre-processing unit 50, a processor 60, a post-processing unit 70 and a display unit 80. Further, the system 100 may include a user interface 101 and a stress sensing unit 102.
The transmit signal forming unit 10 forms transmit signals, which have a pulse form, to form a B-mode ultrasound image of a target-object including reflectors and medium surrounding the reflectors, etc. When stress is not applied to a target object, the transmit signal forming unit 10 forms a first transmit signal. However, when the stress is applied to the target object, the transmit signal forming unit 10 forms a second transmit signal having a different waveform from that of the first transmit signal.
The probe 20 converts the first transmit signal and the second transmit signal into a first ultrasound transmit signal and a second ultrasound transmit signal, respectively. The probe 20 then transmits the first and second ultrasound transmit signals to the target object. The probe 20 also forms receive signals based on the ultrasound echo signals from the target object. That is, the probe 20 forms a first receive signal and a second receive signal corresponding to the first and second transmit signals, respectively.
The frame data forming unit 30 focuses the first or second receive signal to form the frame data. That is, the frame data forming unit 30 forms first frame data by focusing the first receive signal, whereas it forms second frame data by focusing the second receive signal. The frame data may be formed as RF data or baseband complex data.
The storage unit 40 stores the frame data in a frame order. The storage unit 40 stores the first frame data and the second frame data.
The pre-processing unit 50 extracts the first and second receive signals from the first and second frame data, respectively. The pre-processing unit 50 performs log compression to the first and second receive signals to increase the amplitudes of the two signals and to reduce the error caused by noises, thereby obtaining the pre-processed frame data.

The processor 60 computes the strain of the target object and estimates the compressibility of the target object from the pre-processed frame data. Specifically, the processor 60 computes displacement based on the pre-processed first receive signal and the second receive signal by using a cross-correlation method or an auto-correlation method. The processor 60 computes a local displacement, i.e., strain, by differentiating displacement with respect to the distance. Further, the processor 60 selects a maximum value among the computed displacements based on the pre-processed first and second receive signals by using auto-correlation or cross-correlation and then estimates the maximum value as compressibility $\alpha$. Since an elastic coefficient is not constant in the target object having a complicated composition like a human body, the compressibility $\alpha$ changes in every scan line. Thus, the processor 60 computes the compressibility $\alpha$ for every scan line and computes an average compressibility $\alpha_m$. Since the compressibility $\alpha$ is proportional to the stress, the computed average compressibility $\alpha_m$ can be regarded as the average stress in size.

The post-processing unit 70 divides the strain by an average compressibility $\alpha_m$, as shown in the following equation (9), in order to form normalized strains for forming the ultrasound elastic image.

$$\text{Normalized strain} = \text{strain}/\left|\alpha_m\right| \quad \ldots\ldots\ldots\ldots(9)$$

Then, the post-processing unit 70 maps each pixel of the ultrasound elastic image to pseudo color according to the magnitude of the normalized strains. The above-described normalization can compensate for the variation in the stress magnitude. Further, after computing the normalized strain, the post-processing unit 70 may perform low-pass filtering or median filtering for reducing the remaining noises. In the ultrasound elastic image, the contrast varies according to the magnitude of stress, which depends on the velocity of applying stress, skillfulness of the user and the like.

The display unit 80 displays the B-mode ultrasound image based on the frame data provided from the frame data forming unit 30 and the ultrasound elastic image provided from the post-processing unit 70. The display unit 80 may display the B-mode ultrasound image and the ultrasound elastic image at the same time.

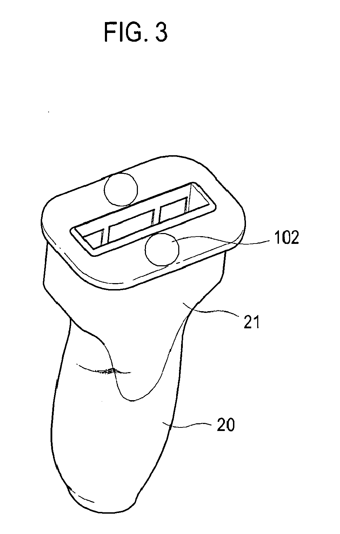

The user interface 101 receives an initial signal from the user when the stress begins to be applied to the target object. The stress sensing unit 102 produces a stress sensing signal by sensing the stress that is applied to the target object through the probe 20. The stress sensing unit 102 may be attached to the probe 20 as shown in FIG. 3. The probe 20 may include a stress transferring part 21, which surrounds a scanning surface of the probe 20, to uniformly apply the stress to the target. In such a case, the stress sensing unit 102 may be attached to the stress transferring part 21.

Hereinafter, a method of forming the first and second transmit signals, which have different waveforms, in the transmit signal forming unit 10 will be described in detail. The transmit signal forming unit 10 forms the second transmit signal in response to the initial signal provided from the user interface 101 or the stress sensing signal provided from the stress sensing unit 102. The second transmit signal is formed based on the compressibility $\alpha$ or the average compressibility $\alpha_m$ provided from the processor 60. That is, the transmit signal forming unit 10 produces a first transmit signal $p_1(t)$, to which the compressibility is not reflected, when the stress is not applied to the target object. Further, the transmit signal forming unit 10 produces a second transmit signal $p_2(t)$, to which the compressibility is reflected, when the stress is applied. In other words, the second transmit signal $p_2(t)$ may be formed by compressing the first transmit signal $p_1(t)$ in the time-axis. The following equation (10) shows a relation between the first transmit signal $p_1(t)$ and the second transmit signal $p_2(t)$

$$p_2(t) = p_1(\alpha t) \quad \ldots\ldots\ldots(10)$$

The following equations (11) and (12) represent the receive signal $r_3(t)$ and the receive signal $r_4(t)$ corresponding to the first transmit signal $p_1(t)$ and the second transmit signal $p_2(t)$, respectively.

$$r_3(t) = p_1(t) * s(t) \quad \ldots\ldots\ldots\ldots(11)$$

$$r_4(t) = p_2(t) * s(t) \qquad \ldots\ldots\ldots\ldots(12)$$

As described above, when the transmit signal is compressed in the time axis by reflecting the compressibility of the target object under the stress, the receive signal $r_4(t)$ is expressed as the following equation (13).

$$r_4(t) = p_1(\alpha t) * s(t) \qquad \ldots\ldots\ldots\ldots(13)$$

The receive signal $r_4(t)$ is extended by the compressibility $\alpha$ in the time axis so as to become equal to the receive signal $r_3(t)$. As the second transmit signal for forming ultrasound signals, which are to be transmitted to the target object under stress, is formed in consideration of the compressibility, the correlation between the receive signal $r_3(t)$ from the target object under no stress and the receive signal $r_4(t)$ from the object under stress increases. Therefore, the strain estimation efficiency is improved. That is, the receive signal $r_3(t)$ and the receive signal $r_4(t)$ in the equations (12) and (13) have a relation as shown in the following equation (14).

$$r_4(t/\alpha) = r_3(t) \qquad \ldots\ldots\ldots\ldots(14)$$

As can be seen from the equation (14), the receive signal $r_4(t)$ from the target object under stress is extended by the compressibility $\alpha$ in the time axis to become equal to the receive signal $r_3(t)$ from the target object under no stress. Thus, the strain can be obtained by applying the compressibility ranging from $\alpha$ to $|1-\alpha|$.

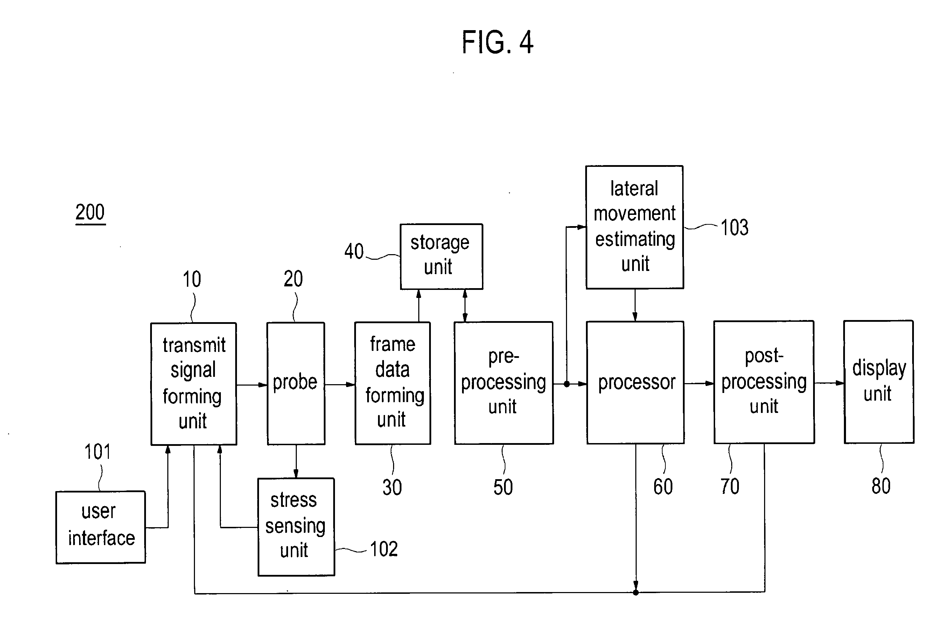

FIG. 4 is a block diagram showing an ultrasound diagnostic system constructed in accordance with a second embodiment of the present invention. The ultrasound diagnostic system 200 further includes a lateral movement estimating unit 103, in addition to the various components described in connection with the ultrasound diagnostic system 100 shown in FIG. 2.

The lateral movement estimating unit 103 receives the pre-processed first and second receive signals and estimates the movement of the target object due to the stress. When stress is applied to a human body, reflectors in the human body move along the lateral direction. This is because the elasticity of medium surrounding the reflectors is not uniform. The lateral movement of the reflectors decreases the correlation between signals and causes a computation error. Thus, the lateral movement is computed from the continuous frame data in order to compensate the error caused by the lateral movement of the reflectors.

The magnitude of lateral movement can be computed with the method of matching speckle patterns in the B-mode image or the correlation between adjacent scan lines in continuous image frames. When the lateral movement is observed in two neighboring frames, a displacement corresponding to the movement is computed.

The processor 60 computes the strain of the target object from the pre-processed frame data based on the displacement provided from the lateral movement estimating unit 103.

The following components of the ultrasound diagnostic system 200 have the same configurations and functions as those of the ultrasound diagnostic system 100 shown in FIG. 2A: a transmit signal forming unit 10; a probe 20; a frame data forming unit 30; a storage unit 40; a pre-processing unit 50; a post-processing unit 70; a display unit 80; a user interface 101; and a stress sensing unit 102. Thus, their detailed descriptions will be omitted herein.

In accordance with the present invention, different transmit signals are transmitted to a target object according to whether stress is applied or not to the target object. Thus, it is possible to improve the correlation between the receive signals, which varies depending on the stress.

Further, compressibility, which varies with scan lines, is used to normalize the ultrasound elastic image to compensate the variation in the stress magnitude, which depends on the user.

An embodiment may be achieved in whole or in part by an ultrasound diagnostic system, which includes: a unit for forming transmit signals, wherein waveforms of the transmit signals vary depending on whether or not stress is applied to a target object; a probe for converting the transmit signals into ultrasound signals and forming receive signals based on ultrasound echo signals reflected from the target object in the presence and absence of stress; a processor for computing a displacement and a strain of the target object due to the stress based on the receive signals; and a post-processing unit for forming an ultrasound elastic image based on the strain.

Any reference in this specification to "one embodiment," "an embodiment," "example embodiment," etc. means that a particular feature, structure or characteristic described in connection with the embodiment is included in at least one embodiment of the invention. The appearances of such phrases in various places in the specification are not necessarily all referring to the same embodiment. Further, when a particular feature, structure or characteristic is described in connection with any embodiment, it falls within the purview of one skilled in the art to effectuate such a feature, structure or characteristic in connection with other ones of the embodiments.

Although embodiments have been described with reference to a number of illustrative embodiments thereof, it should be understood that various other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, drawings and appended claims. In addition to such variations and modifications in the component parts and/or arrangements, alternative uses will also be apparent to those skilled in the art.

**Claims**

1.   An ultrasound diagnostic system for forming an ultrasound elastic image, comprising:

   a unit for forming transmit signals, wherein waveforms of the transmit signals vary depending on whether or not a stress is applied to a target object;
   a probe for converting the transmit signals into ultrasound signals, the probe being configured to form receive signals based on ultrasound echo signals reflected from the target object in the presence and absence of the stress;
   a processor for computing a strain of the target object due to the stress based on the receive signals; and
   a post-processing unit for forming an ultrasound elastic image based on the strain.

2.   An ultrasound diagnostic system for forming an ultrasound elastic image, comprising:

   a unit for forming transmit signals, wherein waveforms of the transmit signals vary depending on whether or not a stress is applied to a target object;
   a probe for converting the transmit signals into ultrasound signals, the probe being configured to form receive signals based on ultrasound echo signals reflected from the target object in the presence and absence of the stress;
   a processor for computing a displacement and a strain of the target object due to the stress based on the receive signals, the processor being configured to estimate a compressibility based on the displacement; and
   a post-processing unit for forming an ultrasound elastic image based on the compressibility and the strain.

3.   An ultrasound diagnostic system for forming an ultrasound elastic image, comprising:

   a unit for forming a first transmit signal to form first ultrasound signals to be transmitted to a target object not being applied with a stress and a second transmit signals to form second ultrasound signals to be transmitted to the target object applied with the stress, wherein the first transmit signal and the second transmit signal have different waveforms;
   a probe for converting the first and second transmit signals into the first and second ultrasound signals, the probe being configured to form first receive signals and second receive signals corresponding to the first transmit signals and the second transmit signals, respectively, based on ultrasound echo signals reflected from the target object;
   a frame data forming unit for forming first frame data and second frame data based on the first receive signals and the second receive signals, respectively;
   a processor for computing a displacement and a strain of the target object due to the stress based on the first receive signals and the second receive signals, the processor being configured to estimate a compressibility based on the displacement; and
   a post-processing unit for forming an ultrasound elastic image based on the compressibility and the strain.

4.   The ultrasound diagnostic system of Claim 3, further comprising a pre-processing unit for extracting the first receive signal and the second receive signal from the first and second frame data, respectively, the pre-processing unit being configured to perform a log compression on the first receive signals and the second receive signals, wherein the processor computes the displacement of the target object due to the stress based on the log-compressed first and second receive signals and computes the strain differentiating the displacement with respect to a distance.

5. The ultrasound diagnostic system of Claim 3, wherein the transmit signal forming unit forms the second transmit signals with application of the compressibility.

6. The ultrasound diagnostic system of Claim 4, further comprising a lateral movement estimating unit for estimating a lateral movement of the target object due to the stress based on the log-compressed first and second receive signals, wherein the processor computes the displacement based on the estimated lateral movement and computes the strain based on the lateral displacement.

7. The ultrasound diagnostic system of Claim 5, wherein the probe includes a plurality of transducer elements, and wherein the processor computes a compressibility for each scan line from each transducer element to the target object and obtains an average compressibility by averaging compressibilities of all scan lines.

8. The ultrasound diagnostic system of Claim 7, wherein the transmit signal forming unit forms the second transmit signals based on the average compressibility.

9. The ultrasound diagnostic system of Claim 5, further comprising a stress sensing unit for sensing the stress applied to the target object to produce a stress sensing signal, wherein the transmit signal forming unit forms the second transmit signals in response to the stress sensing signal.

10. The ultrasound diagnostic system of Claim 5, further comprising a user interface for receiving an initial signal from a user when the stress begins to be applied to the target object, wherein the transmit signal forming unit forms the second transmit signals in response to the initial stress.

11. The ultrasound diagnostic system of Claim 7, wherein the post-processing unit forms the ultrasound elastic image based on a normalized strain obtained by dividing the strain by an absolute value of the average compressibility.

12. A method of forming an ultrasound elastic image, comprising:

forming transmit signals, wherein waveforms of the transmit signals vary depending on whether or not a stress is applied to a target object;
converting the transmit signals into ultrasound signals and forming receive signals based on ultrasound echo signals reflected from the target object in the presence and absence of the stress;
computing a strain of the target object due to the stress based on the receive signals; and
forming an ultrasound elastic image based on the strain.

13. A method of forming an ultrasound elastic image, comprising:

forming transmit signals, wherein waveforms of the transmit signals vary depending on whether or not a stress is applied to a target object;
converting the transmit signals into ultrasound signals and forming receive signals based on ultrasound echo signals reflected from the target object in the presence and absence of the stress;
computing a displacement and a strain of the target object due to the stress based on the receive signals and estimating a compressibility based on the displacement; and
forming an ultrasound elastic image based on the compressibility and the strain.

14. A method of forming an ultrasound elastic image, comprising:

forming a first transmit signal to form first ultrasound signals to be transmitted to a target object not applied with a stress and a second transmit signals to form second ultrasound signals to be transmitted to the target object applied with the stress, wherein the first transmit signal and the second transmit signal have different waveforms;
converting the first and second transmit signals into the first and second ultrasound signals, and forming first receive signals and second receive signals corresponding to the first transmit signals and the second transmit signals, respectively, based on ultrasound echo signals reflected from the target object;
forming first frame data and second frame data based on the first receive signals and the second receive signals, respectively;
computing a displacement and a strain of the target object due to the stress based on the first receive signals and the second receive signals, and estimating a compressibility based on the displacement; and
forming an ultrasound elastic image based on the compressibility and the strain.

15. The method of Claim 14, further comprising:

extracting the first receive signal and the second receive signal from the first and second frame data, respectively, and performing a log compression on the first receive signals and the second receive signals,

wherein the displacement of the target object due to the stress is computed based on the log-compressed first and second receive signals, and wherein the strain is computed by differentiating the displacement with respect to a distance.

16. The method of Claim 14, wherein the second transmit signals are formed with application of the compressibility.

17. The method of Claim 15, further comprising:

estimating a lateral movement of the target object due to the stress based on the log-compressed first and second receive signals, wherein the displacement is computed based on the estimated lateral movement and the strain is computed based on the lateral displacement.

18. The method of Claim 16, wherein the first and second receive signals are obtained from a plurality of transducer elements,
a compressibility for each scan line from each transducer element to the target object is computed, and
an average is obtained from compressibilities of all scan lines.

19. The method of Claim 18, wherein the second transmit signals are formed based on the average compressibility.

20. The method of Claim 16, further comprising:

sensing the stress applied to the target object; and
producing a stress sensing signal,

wherein the second transmit signals are formed in response to the stress sensing signal.

21. The method of Claim 17, further comprising:

receiving an initial signal from a user when the stress begins to be applied to the target object, wherein the second transmit signals are formed in response to the initial stress.

22. The method of Claim 18, wherein the ultrasound elastic image is formed based on a normalized strain obtained by dividing the strain by the average compressibility.

# FIG. 1
# (PRIOR ART)

T

before
compression

after
compression

compression

T/α

**FIG. 2**

FIG. 3

# FIG. 4

EP 1 810 620 A1

European Patent
Office

## EUROPEAN SEARCH REPORT

Application Number

EP 07 00 1377

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 614 388 A (INST NAT SANTE RECH MED [FR]) 11 January 2006 (2006-01-11) * the whole document * | 1-22 | INV. A61B8/08 |
| A | US 6 508 768 B1 (HALL TIMOTHY J [US] ET AL) 21 January 2003 (2003-01-21) * the whole document * | 6,17 | |
| A | US 2002/040187 A1 (ALAM SHEIKH KAISAR [US] ET AL) 4 April 2002 (2002-04-04) * paragraph [0031]; figure 1 * | 6,17 | |
| A | DE 198 24 108 A1 (PESAVENTO ANDREAS [DE]; ERMERT HELMUT [DE]) 2 December 1999 (1999-12-02) * page 5, line 60; figure 3 * | 4,15 | |

TECHNICAL FIELDS
SEARCHED (IPC)

A61B

The present search report has been drawn up for all claims

| Place of search | Date of completion of the search | Examiner |
|---|---|---|
| Berlin | 10 May 2007 | Bernas, Yves |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

                                      
& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                    EP 07 00 1377

This annex lists the patent family members relating to the patent documents cited in  the above-mentioned European search report.
The members are as contained in the European Patent Office EDP file on
The European Patent Office is in no way liable for these particulars which are merely given for the purpose of information.

10-05-2007

| Patent document cited in search report | | Publication date | Patent family member(s) | | Publication date |
|---|---|---|---|---|---|
| EP 1614388 | A | 11-01-2006 | WO | 2006005632 A1 | 19-01-2006 |
| US 6508768 | B1 | 21-01-2003 | NONE | | |
| US 2002040187 | A1 | 04-04-2002 | NONE | | |
| DE 19824108 | A1 | 02-12-1999 | WO | 9961903 A2 | 02-12-1999 |
| | | | EP | 1092148 A2 | 18-04-2001 |
| | | | US | 6520913 B1 | 18-02-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020060007102